# EUROPÄISCHE PATENTSCHRIFT

(11) **EP 1 591 075 B1**
(45) Veröffentlichungstag und Bekanntmachung des Hinweises auf die Patenterteilung: **19.03.2008**
(21) Anmeldenummer: 04009937.6
(22) Anmeldetag: 27.04.2004
(51) Int. Cl.: A61B 19/00

(54) **Planungsverfahren und -vorrichtung für Knieimplantationen**
Method and device for planning knee implants
Procédé et dispositif pour la préparation d'un implant de genou

(43) Veröffentlichungstag der Anmeldung: 02.11.2005
(73) Patentinhaber: BrainLAB AG, 85551 Kirchheim/Heimstetten (DE)
(72) Erfinder: Henning, Stefan, 85570 Markt Schwaben (DE); Hobelsberger, Monika, 85586 Poing (DE); Hächler, Jörg, 85625 Glonn (DE); Slomczykowski, Michal, Harrogate, HG2 9 QG (GB)
(74) Vertreter: Rögner, Jürgen

(56) Entgegenhaltungen:
- WO-A-20/04017836
- WO-A-20/04032780
- DE-A- 10 031 887
- US-A1- 2002 198 451
- US-B1- 6 450 978

## Beschreibung

Die Erfindung betrifft ein Planungsverfahren und eine Planungsvorrichtung für Knieimplantationen. Beim Setzen von Knieimplantaten sollte ein Implantat in einer solchen Weise am verbleibenden Knochen angebracht werden, dass später auch bei der Bewegung keine zu hohen bzw. einseitig zu hohen Belastungen auftreten, die zu einem Schmerzempfinden führen können. Insbesondere möchte man die richtige Positionierung der Patella sicherstellen, um deren Lauf auch bei eingesetztem Implantat optimal gestalten zu können.

Um diese Randbedingungen bei der Planung der Implantation zu berücksichtigen, wird schon herkömmlicherweise ein sogenanntes Patella-Tracking in einer sehr einfachen Ausführung durchgeführt. Wenn im vorliegenden Text von "Tracking" oder "Motion-Capturing" die Rede ist, so ist damit ganz allgemein die Verfolgung der Bewegung eines Körpers mit Hilfe von Bewegungserfassungseinrichtungen gemeint. Dabei können insbesondere ein Körper selbst oder an ihm angebrachte aktive Abstrahler oder passive Reflektoren verfolgt bzw. getrackt werden, um deren Aufenthaltsort zu einem bestimmten Zeitpunkt (prä-/intra-/post operativ) bzw. ihre Bahn zu erfassen und somit auch den Ort oder die Bahn des Körpers bestimmen zu können. Bei einem Ausführungsbeispiel handelt es sich beispielsweise um passive Marker-Reflektoren, die Infrarotlicht zurückstrahlen, welches durch zwei Infrarotlichtkameras erfasst wird, wobei durch die stereoskopische Betrachtung räumliche Orte bzw. räumliche Bewegungen des Körpers bestimmt werden können.

Bei der oben genannten, herkömmlichen Bewegungserfassung für die Patella werden an dieser sowie an dem Femur- und evtl. auch Tibiaknochen Referenzanordnungen (Anordnungen von beispielsweise drei der oben genannten Marker) angebracht, alle drei Knochen werden registriert, und man verfolgt bzw. trackt einen einzigen oder nur wenige einzelne Punkte der Patella, um deren Bahn gegenüber dem Femurknochen zu erfassen.

Der Nachteil dieser bekannten Verfahren liegt einfach in ihrer geringen Aussagekraft über die später zu erwartenden relativen Bewegungen und Berührungen der einzelnen Gelenkteile. Das punktweise Tracking bzw. Motion-Capturing für einzelne Punkte der Patella ist unzureichend für eine realitätsnahe Simulation der Endbedingungen und damit auch nicht ausreichend für eine optimale Planung.

Es ist die Aufgabe der vorliegenden Erfindung, ein Planungsverfahren und eine Planungsvorrichtung für Knieimplantationen vorzustellen, welche die vorgenannten Nachteile des Standes der Technik überwinden. Insbesondere soll ein Verfahren bereitgestellt werden, welches eine optimale Planung ermöglicht und somit auch hinsichtlich der Lage und Bahn der Patella nach dem setzen des Implantats für günstige Verhältnisse sorgt.

Diese Aufgabe wird durch ein Planungsverfahren für Knieimplantationen gelöst, wie es im Anspruch 1 beschrieben ist, sowie durch eine Vorrichtung gemäß Anspruch 11. Die Unteransprüche beschreiben bevorzugte Ausführungsformen der Erfindung.

Die Vorteile der vorliegenden Erfindung beruhen darauf, dass die Patienten-Knieanatomie räumlich bzw. der Oberflächenkontur nach erfasst wird, so dass Berührungen oder Überschneidungen zwischen Implantat und den Teilen der Knieanatomie sehr viel umfänglicher und mit sehr viel höherer Aussagekraft erfasst und simuliert werden können. Auf der Basis dieser Ergebnisse lässt sich dann sehr viel präziser als bisher ein mögliches Problem bei einer momentan vorgeschlagenen Implantatpositionierung erkennen, und die Positionierung lässt sich anpassen, bis das Problem nicht mehr auftritt.

Mit anderen Worten ist die Erfindung auch ein Teil der Patella-Tracking-Funktionalität eines chirurgischen computergestützten Planungssystems. Die allgemeine Idee liegt auch darin, die Bewegung der Patella zum Femurknochen hin zu verfolgen, wobei als postoperatives Resultat die Patella oder ein Patellaimplantat in einer solchen Weise am Femur oder an dem Femurimplantat gleiten sollte, dass der Patient am hinteren oder vorderen Teil der Patella keinen Schmerz verspürt. Dieses Resultat kann dadurch erzielt werden, dass sichergestellt wird, dass die Patella nach der Implantation anatomisch läuft und keine unnatürlichen Belastungen auf die Patella aufgebracht werden. Das gilt auch wenn die Patella-Gleitfläche durch ein Implantat ersetzt wurde.

Gemäß einer bevorzugten Ausführungsform des erfindungsgemäßen Verfahrens werden die räumlichen Daten der Gestalt der Patienten-Knieanatomie durch eine Oberflächen-Abtastung mittels eines getrackten Abtastinstruments erfasst, insbesondere mittels eines navigierten Pointers eines chirurgischen Navigationssystems. Eine solche Ausführungsform gestaltet das gesamte Umfeld sehr einfach und übersichtlich und macht die Verwendung komplizierter und teurer Apparate für die Oberflächen-Abtastung überflüssig. Von besonderem Vorteil ist es bei solchen Abtastungen, wenn diejenigen Flächen der Patienten-Knieanatomie abgetastet bzw. erfasst werden, welche sich später auch berühren bzw. aneinander laufen oder Kontakt haben, also die Kontakt- bzw. Laufflächen der Knie-Anatomieteile. Es kann aber auch die Oberflächenabtastung erst erfolgen, wenn bereits eine vollständige Bewegung des Beins durchgeführt wurde und dabei lediglich die relative Bewegung zwischen den bereits angebrachten Referenzsystemen aufgezeichnet wurde. Dieses Vorgehen wird weiter unten noch genauer erklärt.

Es soll im Rahmen der vorliegenden Erfindung aber nicht völlig ausgeschlossen werden, dass auch hochgenaue Oberflächen-Abtastungen mit technischer Hilfe durchgeführt werden. So ist es gemäß einer anderen Ausführungsform der vorliegenden Erfindung möglich, die räumlichen Daten der Gestalt der Patienten-Knieanatomie durch eine Oberflächen-Abtastungen mit Hilfe eines Schichtbild-Erfassungsverfahrens, insbesondere eines CT-Scans zu erfassen.

Die genannten Abtastungen können prä-operativ oder intraoperativ erfolgen. Die Bewegungsaufzeichnung kann aber auch post-operativ erfolgen, wenn beispielsweise ein implantierter Referenzmarker in den genannten Knochen des Patienten verbleibt und z.B. in einem magnetischen Feld vermessen wird. Ist die Position des Referenzmarkers zum Knochenbild bekannt, wie beispielsweise durch eine intraoperativ durchgeführte Referenzierung, kann die aufgezeichnete Bewegung mit den prä- bzw. intraoperativ aufgezeichneten Bewegungen verglichen und analysiert werden.

Bei einer Ausführungsform der Erfindung werden die Berührungen oder Überschneidungen dann als unkritisch angesehen, wenn sie im Wesentlichen symmetrische Kontakte zwischen den Teilen der Knieanatomie bzw. eine Vermeidung von Patella-Alta oder Patella-Bacha indizieren.

Die Anpassung der Positionierung, der Form oder Ausrichtung des Implantats kann erfindungsgemäß automatisch durch das computergestützte Planungssystem erfolgen. Dies ist aber nicht die einzige Möglichkeit. Vielmehr ist es erfindungsgemäß auch möglich, das computergestützte Planungssystem Daten über mindestens eine mögliche Anpassung ausgeben zu lassen, wobei die geeignete Anpassung dann durch Bedienereingaben bestätigt oder ausgewählt werden kann. Eine dritte Möglichkeit ist dann eine Anpassung durch den Bediener auf der Basis der erfassten Berührungs- oder Überschneidungsdaten.

Die erfassten Berührungs- oder Überschneidungsdaten an den Teilen der Knieanatomie bzw. an den Implantaten (dem Implantat) werden gemäß einer Ausführungsvariante des erfindungsgemäßen Verfahren in einer Bildausgabe dargestellt. Diese Bildausgabe kann enthalten:
- die Patellaoberfläche,
- die Patella-(Probe)Implantat-Oberfläche,
- den distalen Femurknochen bzw. die Gelenkhöcker ("Kondylen"),
- das Femurimplantat,
- den proximalen Tibiaknochen
- das Tibiaimplantat und Insert/Inlet
- Querschnittsansichten durch Oberflächen- oder Volumendarstellung der oben genannten Punkte
- Virtuell dargestellte Bänder oder Bandansatzpunkte

Einzelne oder mehrere dieser dargestellten Körper können in der Bildausgabe enthalten sein. Dabei sind die unterschiedlichsten Darstellungsmöglichkeiten denkbar, beispielsweise können die erfassten Berührungs- oder Überschneidungsdaten einfarbig, einfarbig mit unterschiedlichen Schattierungen oder unterschiedlichen Farben, abhängig von der Intensität der Berührung bzw.

Überschneidung dargestellt werden. Wichtig ist, dass die Berührungs- oder Überschneidungsdaten bzw. die Kontaktdaten gut unterscheidbar aufgezeigt werden. Ferner besteht erfindungsgemäß die Möglichkeit, die erfassten Berührungs- oder Überschneidungsdaten an den Teilen der Knieanatomie bzw. an den Implantaten als Text/Zahlenausgabe oder als zusätzliche Text/Zahlenausgabe in einer Bildausgabe darzustellen. Grundsätzlich ist natürlich auch lediglich eine Text/Zahlenausgabe denkbar.

Insbesondere eignet sich das erfindungsgemäße Verfahren auch in solchen Fällen, wo Bandkorrekturen erfolgen. Besonders in den Fällen, wo solche Bandkorrekturen einen erheblichen Einfluss auf die Kniebewegung insgesamt oder die Bahn der Patella im Speziellen haben, kann die Veränderung der Patellabewegung nach einer Knieband-Längenkorrektur bestimmt und wahlweise bei der Anpassung berücksichtigt werden.

Die Erfindung wird im Weiteren detaillierter beschrieben, wobei auf spezielle Ausführungsformen und die beiliegenden Zeichnungen Bezug genommen wird. Es zeigen:
- Figur 1: einen Screen-Shot eines Planungssystems zur computergestützten Implantationsplanung, auf dem die Stärke der Berührung zwischen verschiedenen Knie-Teilen bzw. Implantaten dargestellt wird; und
- Figur 2: einen Screen-Shot mit einer Darstellung der Patellaverschiebung bei einer Kniebandbehandlung.

Bei dem Planungsverfahren gemäß der vorliegenden Erfindung wird anfangs die Bewegung der Patella im ursprünglichen Zustand des Kniegelenkes aufgezeichnet, wenn das Knie gebeugt und gestreckt wird. Dies kann prä- und/oder intraoperativ geschehen, wobei an der Patella sowie an den Gelenkenden von Femur und Tibia Referenzmarkeranordnungen angebracht und durch ein beispielsweise kameragestütztes oder magnetisches Trackingsystem verfolgt werden. Vor oder nach diesem "Motion-Capturing" werden diejenigen Oberflächen von Patella, Femur und Tibia, speziell der Patella und des Femurknochens, die bei der Kniebewegung miteinander in Kontakt kommen bzw. aneinander laufen, der äußeren Form bzw.

Gestalt nach erfasst. Dies geschieht hier beispielsweise dadurch, dass die Oberflächen mit der Spitze eines ebenfalls getrackten Abtastinstruments abgefahren werden, so dass im Planungssystem die äußere Kontur flächenmäßig als Datensatz abgelegt werden kann. Nunmehr kennt das Planungssystem den natürlichen Bewegungsablauf zwischen Patella und den restlichen Gelenkteilen untereinander, und es kennt die Gestalt der einander berührenden und aufeinander laufenden Oberflächen in diesem anatomischen Zustand.

Das Planungssystem umfasst auch Daten für Knieimplantate, insbesondere Daten über die Form solcher Knieimplantate. Wenn nunmehr an der Planungsstation virtuell ein Teil der Knieanatomie, beispielsweise des Femur-Gelenkkopfes, durch ein solches Knieimplantat ersetzt wird, z.B. durch Überlagerung der geplanten Femurkomponente auf die Patellakinematik, können die Kontakt- oder Laufflächen anhand der entstehenden Berührungen oder Überschneidungen errechnet und in einer Bilddarstellung hervorgehoben werden, so dass der Chirurg Informationen darüber erhält, wie die Position des Implantats die Spannung und die Kinematik der Patella beeinflusst. Mit anderen Worten wird die aufgezeichnete Bewegung der Patella beispielsweise in einer Implantatpositionierungs-Planungsansicht im Bezug auf den Femurknochen und eine geplante Femur-Implantatposition dargestellt.

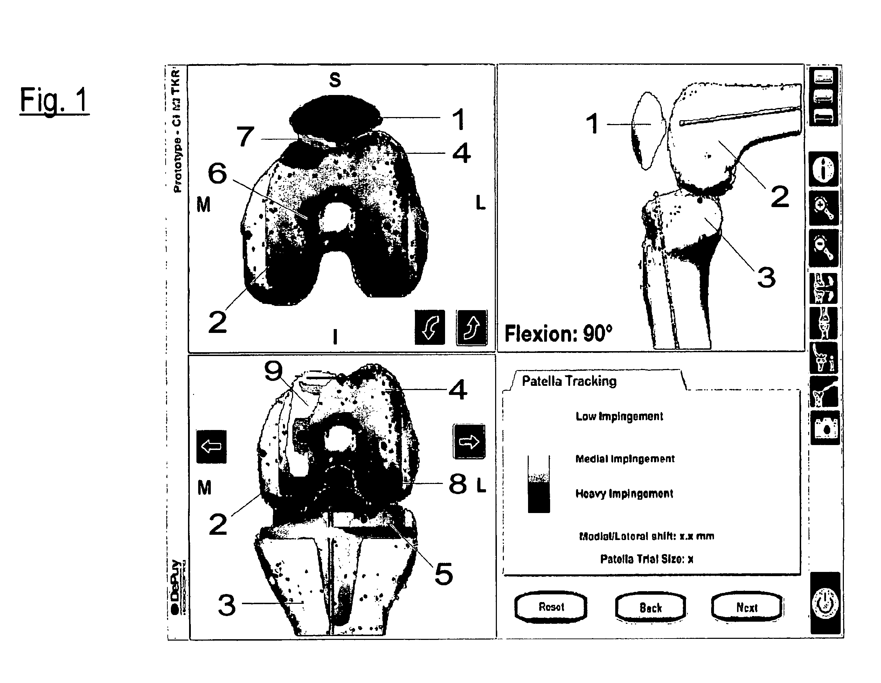

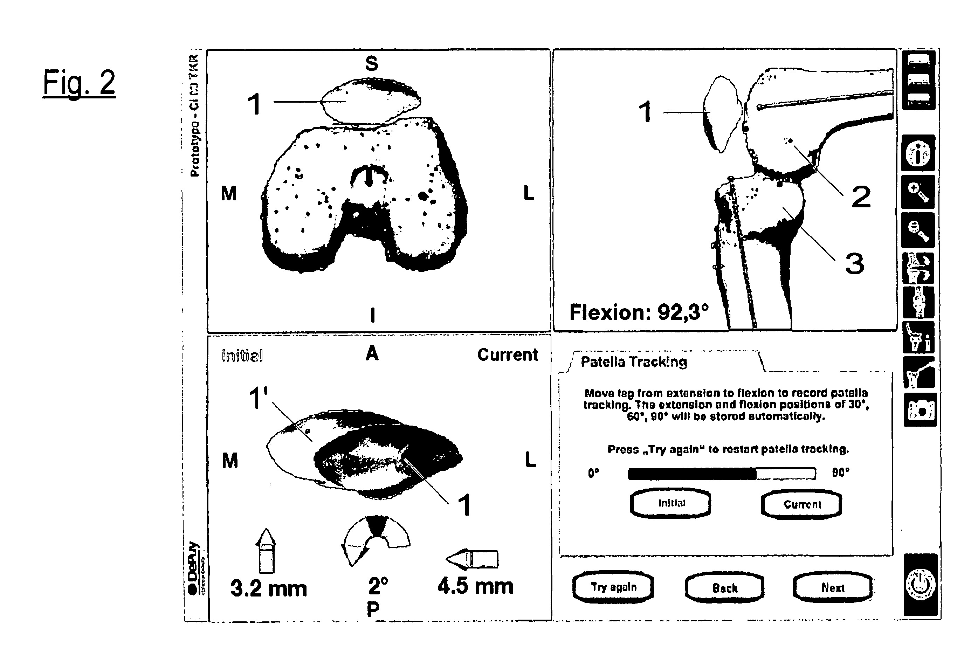

Es ergibt sich beispielsweise eine Darstellung gemäß Figur 1. In Figur 1 (und in Figur 2) bezeichnet das Bezugszeichen 1 die Patella, das Bezugszeichen 2 den Femurknochen und das Bezugszeichen 3 die Tibia. In beiden Figuren ist rechts oben eine Winkelstellung des Kniegelenkes in einer Seitenansicht dargestellt, darunter jeweils ein Ein-/Ausgabefeld der Software. Links oben zeigt die Darstellung den Femurknochen mit Patella und in Figur 1 links unten sind Femur und Tibia mit Implantaten gezeigt. Im Bild rechts oben kann auch das Femurimplantat mit dargestellt werden.

Links oben in Figur 1 ist die Position der Patella 1 über dem Femurknochen 2 dargestellt. Auf dem Femur 2 ist ein Implantat 4 angeordnet. Auf dem Implantat 4 sowie an der Patella ist durch unterschiedliche Schattierung angedeutet, wo ein starker Kontakt (Impingement) und wo ein schwächerer Kontakt stattfindet, wenn das Knie mit dem gesetzten Implantat bewegt wird. Die dunkleren Flächen weisen, wie in der Legende rechts unten aufgezeigt ist, eine stärkere Berührung bzw. einen stärkeren Kontakt auf als die helleren Stellen; eine dunkle Stelle am Femurimplantat ist mit dem Bezugszeichen 6, eine helle Stelle an der Patella mit dem Bezugszeichen 7 versehen.

Eine ähnliche Aussage gibt die Darstellung links unten für Femur- und Tibiaimplantat 4 und 5. An der sehr hellen Stelle 9 am Femurimplantat besteht im simulierten Bewegungsablauf wenig oder kein Kontakt, während an der relativ dunklen Stelle 8 stärkerer Kontakt vorliegt.

Auf der Basis dieser Informationen kann nun die Positionierung des Implantats virtuell angepasst werden, beispielsweise durch ein virtuelles Versetzen nach anterior oder posterior, medial oder lateral, oder in einer Rotation. Dies kann automatisch aufgrund der Kontaktstärkeinformationen durch das Planungssystem durchgeführt werden oder "per Hand" durch Benutzereingriff. Am Ende wird man eine Implantatposition finden, bei der die Kontaktintensitäten in einem gewünschten Bereich liegen.

Die Kontakintensität kann hier z.B. einem Druck und/oder der Richtung des Drucks auf die Patella entsprechen. Die Intensität kann z.B. punktuell, über eine Fläche oder volumenbezogen ermittelt werden.

Die Figur 2 zeigt Darstellungen für eine spezielle Anwendung im Rahmen der vorliegenden Erfindung. Wenn nämlich während der Operation eine Änderung der Kinematik bzw. Beinachse, insbesondere z. B. eine Verlängerung der Kniebänder, vorgenommen wird, ist auch zu erwarten, dass die Laufbahn der Patella sich ändert. Um diese Positionsänderungen der Patella zu berücksichtigen, kann der Verwender des erfindungsgemäßen Systems nach den Änderungen an den Bändern nochmals die Bewegung der Teile der Knieanatomie mittels des Tracking- bzw. Motion-Capturingverfahrens aufzeichnen und diese Daten der Planungsstation zur Verfügung stellen. Wenn dann nochmals ein Bewegungsablauf simuliert wird, wird ersichtlich, wie sich die Lage und Bahn der Patella ändert, was in Figur 2 links unten dargestellt ist, wobei das Bezugszeichen 1' die ursprüngliche und das Bezugszeichen 1 die neue Lage der Patella nach der Bandverlängerung aufzeigt.

Unter den beiden Patella-Darstellungen ist auch noch durch Pfeile und Text angegeben, wie sich die Lage genau geändert hat, also beispielsweise eine Verschiebung um 4,5 mm nach links, um 3,2 mm nach oben und eine Drehung um 2° entgegen des Uhrzeigersinns.

Im rechts unten dargestellten Auswahlfenster kann der Bediener dann wählen, ob er die anfängliche oder die derzeitige, neue Bahn als Grundlage für die Implantatpositionierung wählen möchte. Dann wird wiederum die Kontaktintensität dargestellt (siehe Figur 1) und die optimale Implantatposition wird ermittelt.

Nachdem nun "virtuell" die optimale Positionierung des Implantats festgestellt wurde, ist das Planungsverfahren abgeschlossen. Mit den ermittelten Planungsdaten lässt sich die Positionierung einer Vorrichtung (Lehre) bestimmen, mit deren Hilfe die Knochen so abgetragen werden, dass das tatsächliche Implantat in der optimalen Position aufgesetzt und befestigt werden kann.

Die Bewegungsaufzeichnung kann noch einmal post-operativ erfolgen, wenn beispielsweise ein implantierter Referenzmarker in den genannten Knochen des Patienten verbleibt und z.B. in einem magnetischen Feld vermessen wird. Ist die Position des Referenzmarkers zum Knochenbild bekannt, wie beispielsweise durch eine intraoperativ durchgeführte Referenzierung, kann die aufgezeichnete Bewegung mit dem prä- bzw. intraoperativ aufgezeichneten Bewegungen verglichen und analysiert werden.

## Patentansprüche

1. Planungsverfahren für Knieimplantationen, bei dem
- räumliche Daten bzw, Oberflächenkonturdaten der Gestalt einer Patienten-Knieanatomie, nämlich mindestens eines Teils des Femurknochens und/oder der Tibia und der Patella, zur Eingabe in eine computergestützte Planungsstation erfasst werden,
- die Bewegung der Teile der Knie-Anatomie mittels eines Tracking- bzw. Motion-Capturing-Verfahrens aufgezeichnet wird,
- die erfassten Anatomie- und Bewegungsdaten der computergestützten Planungsstation zur Verfügung gestellt werden,
- die Bewegung der einzelnen Teile zueinander auf einem Bildschirm, insbesondere gleichzeitig, dargestellt und/oder der bereits aufgezeichnete Weg nachgestellt wird,
- ein Teil der Patienten-Knieanatomie virtuell in der Planungsstation durch ein Probe-Implantat an Femur und/oder Tibia und Patella ersetzt und Kniebewegungen mit den Probe-Implantaten simuliert werden,
- Berührungen oder Überschneidungen zwischen den nicht ersetzten Teilen der Knieanatomie und mehreren virtuell dargestellten Implantaten bei der virtuellen Bewegung der Größe nach erfasst werden, und bei dem
- eine Anpassung der Positionierung, der Form oder der Ausrichtung der Implantate, oder mehrerer dieser Parameter, bestimmt wird, wenn Berührungen oder Überschneidungen unkritisch werden und die derart bestimmte Anpassung als geeignete Anpassung definiert wird.

2. Planungsverfahren nach Anspruch 1, bei dem die räumlichen Daten der Gestalt der Patienten-Knieanatomie durch eine Oberflächen-Abtastung mittels eines getrackten Abtastinstruments, insbesondere mittels eines navigierten Pointers eines chirurgischen Navigationssystems, erfasst werden.

3. Planungsverfahren nach Anspruch 1, bei dem die räumlichen Daten der Gestalt der Patienten-Knieanatomie durch eine Oberflächen-Abtastung mit Hilfe eines Schichtbild-Erfassungsverfahrens, insbesondere eines CT-Scans erfasst werden.

4. Planungsverfahren nach einem der Ansprüche 1 bis 3, bei dem die Berührungen oder Überschneidungen dann als unkritisch angesehen werden, wenn sie im wesentlichen symmetrische Kontakte zwischen den Teilen der Knieanatomie bzw. eine Vermeidung von Patella Alta oder Patella Bacha indizieren.

5. Planungsverfahren nach einem der Ansprüche 1 bis 4, bei dem die Anpassung automatisch durch das computergestützte Planungssystem erfolgt.

6. Planungsverfahren nach einem der Ansprüche 1 bis 4, bei dem das computergestützte Planungssystem Daten über mindestens eine mögliche Anpassung ausgibt, wobei die geeignete Anpassung durch Bedienereingaben bestätigt oder ausgewählt werden kann.

7. Planungsverfahren nach einem der Ansprüche 1 bis 4, bei dem die Anpassung durch den Bediener auf der Basis der erfassten Berührungs- oder Überschneidungsdaten erfolgt.

8. Planungsverfahren nach einem der Ansprüche 1 bis 7, bei dem die erfassten Berührungs- oder Überschneidungsdaten an den Teilen der Knieanatomie bzw. an den Implantaten in einer Bildausgabe dargestellt werden; wobei die Darstellung enthalten kann:
- die Patellaoberfläche,
- die Patella-Probeimplant-Oberfläche,
- den distalen Femurknochen bzw. die Gelenkhöcker (Kondylen),
- das Femurimplantat,
- den proximalen Tibiaknochen,
- das Tibiaimplantat und Insert/Inlet,
- Querschnittsansichten durch Oberflächen- oder Volumendarstellung der oben genannten Punkte,
- virtuell dargestellte Bänder oder Bandansatzpunkte.

9. Planungsverfahren nach einem der Anspruch 8, bei dem die erfassten Berührungs- oder Überschneidungsdaten einfarbig, einfarbig mit unterschiedlichen Schattierungen oder in unterschiedlichen Farben, abhängig von der Intensität der Berührung bzw. Überschneidung dargestellt werden.

10. Planungsverfahren nach einem der Ansprüche 1 bis 9, bei dem die erfassten Berührungs- oder Überschneidungsdaten an den Teilen der Knieanatomie bzw. an den Implantaten als Text/Zahlenausgabe oder als zusätzliche Text/Zahlenausgabe in einer Bildausgabe dargestellt werden.

11. Vorrichtung zur Planung von Knieimplantationen, mit
- einer computergestützten Planungsstation, in welche räumliche Daten bzw. Oberflächenkonturdaten der Gestalt einer Patienten-Knieanatomie, nämlich eines Teils des Femurknochens und/oder der Tibia und der Patella eingegeben werden,
- einer Einrichtung zur Aufzeichnung der Bewegung der Teile der Knieanatomie mittels eines Tracking- bzw. Motion-Capturingverfahrens,
- einer Einrichtung zur Übertragung der Anatomie- und Bewegungsdaten zur computergestützten Planungsstation, wobei in
- einer Datenverarbeitungseinheit in der Planungsstation, ein Teil der Patienten-Knieanatomie virtuell durch ein Probeimplantat an Femur und/oder Tibia und Patella ersetzt und Kniebewegungen mit den Probe-Implantaten simuliert werden und Berührungen oder Überschneidungen zwischen den nicht ersetzten Teilen der Knieanatomie und den Implantaten bei der virtuellen Bewegung der Größe nach erfasst werden, wobei mit der Datenverarbeitungseinheit eine Anpassung der Positionierung, der Form oder Ausrichtung der Implantate, oder mehrerer dieser Parameter, bestimmt wird, wenn die Berührungen oder Überschneidungen unkritisch werden und derart bestimmte Anpassung als geeignete Anpassung definiert wird.

12. Programm, das, wenn es auf einem Computer läuft oder in einem Computer geladen ist, den Computer veranlasst, ein Verfahren gemäß einem der Ansprüche 1 bis 10 durchzuführen.

13. Computerprogramm-Speichermedium, das ein Programm nach Anspruch 12 aufweist.

## Claims

1. A planning method for knee implants, wherein:
- spatial data and/or surface contour data on the configuration of a patient's genicular anatomy, namely of at least a part of the femur and/or the tibia and the patella, are captured in order to be inputted into a computer-assisted planning station; ,
- the movement of the parts of the genicular anatomy is recorded using a tracking and/or motion capturing method;
- the captured anatomical and movement data are made available to the computer-assisted planning station;
- the movement of the individual parts with respect to each other is displayed on a screen, in particular simultaneously, and/or the path already recorded is subsequently displayed;
- a part of the patient's genicular anatomy is virtually replaced in the planning station by a sample implant on the femur and/or tibia and patella, and movements of the knee together with the sample implants are simulated;
- contact or impingement between the non-replaced parts of the genicular anatomy and a number of virtually displayed implants during the virtual movement is ascertained according to its magnitude; and wherein
- an adjustment of the positioning, shape or orientation of the implants or of a number of these parameters is determined when contact or impingement become non-critical and the adjustment thus determined is defined as a suitable adjustment.

2. The planning method according to claim 1, wherein the spatial data on the configuration of the patient's genicular anatomy are captured by surface scanning using a tracked scanning instrument, in particular a navigated pointer of a surgical navigation system.

3. The planning method according to claim 1, wherein the spatial data on the configuration of the patient's genicular anatomy are captured by surface scanning with the aid of a tomographic imaging method, in particular a CT scan.

4. The planning method according to any one of claims 1 to 3, wherein the contact or impingement is regarded as non-critical if it indicates substantially symmetrical contacts between the parts of the genicular anatomy and/or an avoidance of the patella alta or the patella bacha.

5. The planning method according to any one of claims 1 to 4, wherein the adjustment is automatically performed using the computer-assisted planning system.

6. The planning method according to any one of claims 1 to 4, wherein the computer-assisted planning system outputs data on at least one possible adjustment, wherein the suitable adjustment can be confirmed or selected by user inputs.

7. The planning method according to any one of claims 1 to 4, wherein the adjustment is performed by the user on the basis of the captured contact or impingement data.

8. The planning method according to any one of claims 1 to 7, wherein the captured contact or impingement data on the parts of the genicular anatomy and/or on the implants are displayed in an image output, wherein the representation can include:
- the surface of the patella;
- the surface of the patella sample implant;
- the distal femur and/or tubercula articulare (condyles);
- the femur implant;
- the proximal tibia;
- the tibia implant and insert/inlet;
- cross-sectional views through a surface or volume representation of the points cited above;
- virtually displayed ligaments or ligament attachment points.

9. The planning method according to claim 8, wherein the captured contact or impingement data are displayed in monotone, monotone with different shadings, or in different colours, depending on the intensity of the contact and/or impingement.

10. The planning method according to any one of claims 1 to 9, wherein the captured contact or impingement data on the parts of the genicular anatomy and/or on the implants are displayed as a textual/numerical output or as an additional textual/numerical output in an image output.

11. A device for planning knee implants, comprising:
- a computer-assisted planning station, into which spatial data and/or surface contour data on the configuration of a patient's genicular anatomy, namely of a part of the femur and/or the tibia and the patella, are inputted;
- a means for recording the movement of the parts of the genicular anatomy using a tracking and/or motion capturing method;
- a means for transmitting the anatomical and movement data to the computer-assisted planning station; wherein in
- a data processing unit in the planning station, a part of the patient's genicular anatomy is virtually replaced by a sample implant on the femur and/or tibia and patella, and movements of the knee together with the sample implants are simulated, and contact or impingement between the non-replaced parts of the genicular anatomy and the implants during the virtual movement is detected according to its magnitude, wherein using the data processing unit, an adjustment of the positioning, shape or orientation of the implants or of a number of these parameters is determined when the contact or impingement become non-critical and the adjustment thus determined is defined as a suitable adjustment.

12. A program which, when it is running on a computer or is loaded onto a computer, causes the computer to perform a method in accordance with any one of claims 1 to 10.

13. A computer program storage medium comprising a program according to claim 12.

## Revendications

1. Procédé de conception pour implants du genou, dans lequel
- on relève des données spatiales ou des données du contour superficiel de la forme d'une anatomie du genou d'un patient, à savoir d'au moins une partie de l'os du fémur et/ou du tibia et de la rotule, pour les introduire dans un poste de conception assisté par ordinateur,
- on enregistre le mouvement des parties de l'anatomie du genou au moyen d'un procédé de poursuite ou de captage du mouvement,
- on met les données relevées d'anatomie et de mouvement à disposition du poste de conception assisté par ordinateur,
- on représente le mouvement des différentes parties les unes par rapport aux autres sur un écran, en particulier simultanément et/ou on corrige le trajet déjà enregistré,
- on remplace une partie de l'anatomie du genou du patient de manière virtuelle dans le poste de programmation, par un implant d'essai sur le fémur et/ou le tibia et la rotule, et on simule des mouvements du genou avec l'implant d'essai,
- on relève des contacts ou des recoupements entre les parties non remplacées de l'anatomie du genou et plusieurs implants représentés de manière virtuelle, lors du mouvement virtuel, suivant la taille, et dans lequel
- on détermine une adaptation du positionnement, de la forme ou de l'orientation de l'implant ou de plusieurs de ces paramètres, lorsque les contacts ou recoupements deviennent non critiques, et l'adaptation ainsi déterminée est définie comme adaptation convenable.

2. Procédé de conception selon la revendication 1, dans lequel on relève les données spatiales de la forme de l'anatomie du genou du patient par un balayage de surface au moyen d'un instrument de balayage suivi, en particulier au moyen d'un index navigué d'un système de navigation chirurgical.

3. Procédé de conception selon la revendication 1, dans lequel on relève les données spatiales de la forme de l'anatomie du genou d'un patient par un balayage de surface à l'aide d'un procédé de relevé tomographique, en particulier d'une tomographie par ordinateur.

4. Procédé de conception selon l'une quelconque des revendications 1 à 3, dans lequel on considère les contacts ou recoupements comme non critiques lorsqu'ils indiquent pour l'essentiel des contacts symétriques entre les parties de l'anatomie du genou ou qu'une rotule haute ou une rotule basse sont évitées.

5. Procédé de conception selon l'une quelconque des revendications 1 à 4, dans lequel l'adaptation s'effectue automatiquement par le système de conception assisté par ordinateur.

6. Procédé de conception selon l'une quelconque des revendications 1 à 4, dans lequel le système de conception assisté par ordinateur délivre des données relatives à au moins une adaptation possible, l'adaptation convenable pouvant être confirmée ou choisie par des saisies de l'opérateur.

7. Procédé de conception selon l'une quelconque des revendications 1 à 4, dans lequel l'adaptation est assurée par l'opérateur sur la base des données relevées de contact ou de recoupement.

8. Procédé de conception selon l'une quelconque des revendications 1 à 7, dans lequel les données relevées de contact ou de recoupement dans les parties de l'anatomie du genou ou les implants sont représentées dans une sortie image, la représentation pouvant contenir :
- la surface de la rotule,
- la surface rotule-implant d'essai,
- l'os distal du fémur ou les condyles,
- l'implant du fémur,
- l'os proximal du tibia,
- l'implant du tibia et l'insert/inlet,
- des vues en coupe transversale de surfaces ou la représentation en trois dimensions des points mentionnés ci-dessus,
- des ligaments représentés de manière virtuelle ou des points de naissance de ligaments.

9. Procédé de conception selon la revendication 8, dans lequel les données relevées de contact ou de recoupement sont représentées par une seule couleur avec des nuances différentes ou dans différentes couleurs, en fonction de l'intensité du contact ou du recoupement.

10. Procédé de conception selon l'une quelconque des revendications 1 à 9, dans lequel les données relevées de contact ou de recoupement dans les parties de l'anatomie du genou ou les implants sont représentées sous la forme d'une sortie texte/chiffres ou sous la forme d'une sortie supplémentaire texte/chiffres dans une sortie image.

11. Dispositif pour concevoir des implants du genou, comportant
- un poste de conception assisté par ordinateur dans lequel on introduit des données spatiales ou des données de contour superficiel de la forme d'une anatomie du genou d'un patient, à savoir d'une partie de l'os du fémur et/ou du tibia et de la rotule,
- un dispositif pour enregistrer le mouvement des parties de l'anatomie du genou au moyen d'un procédé de poursuite ou de captage de mouvement,
- un dispositif pour transmettre les données anatomiques et données de mouvement au poste de conception assisté par ordinateur, dans lequel on remplace, dans une unité de traitement de données dans le poste de conception, une partie de l'anatomie du genou d'un patient, de manière virtuelle, par un implant d'essai sur le fémur et/ou le tibia et/ou la rotule, et on simule des mouvements du genou avec les implants d'essai, et on relève des contacts ou des recoupements entre les parties non remplacées de l'anatomie du genou et les implants, lors du mouvement virtuel, suivant la taille, dans lequel, avec l'unité de traitement de données, on détermine une adaptation du positionnement, de la forme ou de l'orientation des implants, ou de plusieurs de ces paramètres, lorsque les contacts ou recoupements deviennent non critiques et on définit l'adaptation ainsi déterminée comme adaptation convenable.

12. Programme qui, lorsqu'il tourne sur un ordinateur ou est chargé dans un ordinateur, fait que l'ordinateur exécute un procédé selon l'une quelconque des revendications 1 à 10.

13. Support d'enregistrement de programme d'ordinateur qui comporte un programme selon la revendication 12.
